# EUROPEAN PATENT SPECIFICATION

(11) **EP 1 542 643 B1**
(45) Date of publication and mention of the grant of the patent: **21.10.2009**
(21) Application number: 03766120.4
(22) Date of filing: 05.08.2003
(51) Int. Cl.: A61H 39/00

(54) **ATMOSPHERIC ELECTRIC ACUPUNCTURE MONITOR**
ATMOSPHÄRISCHER ELEKTRISCHER AKUPUNKTUR-MONITOR
DISPOSITIF DE SURVEILLANCE D'ACUPONCTURE UTILISANT L'ELECTRICITE ATMOSPHERIQUE

(30) Priority: 05.08.2002 DK 200201178
(43) Date of publication of application: 22.06.2005
(73) Proprietor: Wetling, John F., 3390 Hundested (DK)
(72) Inventor: Wetling, John F., 3390 Hundested (DK)
(74) Representative: Inspicos A/S
(86) International application number: PCT/DK2003/000523
(87) International publication number: WO 2004/012645

(56) References cited:
- EP-A1- 0 502 501
- FR-A1- 2 255 922

## Description

### The area of application for the invention

The invention concerns an atmospheric electric acupuncture monitor for producing and regulating an ion current. The regulated current may be used in an acupuncture treatment of human beings as well as animals. The effect of the treatment is caused by the current from the neutralized ions.

### State of the technique

For centuries acupuncture has been used in many parts of the world in the treatment of widely different diseases and ailments. And often with remarkable results. Up till now the treatment has in principle consisted in the subcutaneous insertion of metal needles at certain points of the skin (acupoints) in connection with the so-called meridians. No satisfactory explanation has ever been given as to why such a process should have a physiological (or other type of) effect. And it has been characteristic for acupuncture treatments that the results were often very varying and unpredictable.

It is now the claim of this invention that the effect of acupuncture basically is caused by weak electrical currents caused by the discharge on the skin of atmospheric ions (air ions).
This phenomenon is explained in the following paper by dr. Niels Jonassen: Is *Acupuncture An Electrical Phenomenon.*

### Is Acupuncture an Electrical Phenomenon? by Niels Jonassen, D.Sc.

For centuries acupuncture, the subcutaneous insertion of metallic needles in certain parts of (mostly) the human body, has been practiced in various parts of the world for diagnostic as well as remedial purposes.

There have been plenty of reports of surprising effects of acupuncture on many types of diseases or discomfort, but it should be stressed that these reports are normally anecdotal in character, often collections of single cases, and rarely based on strict scientific investigations, including double blind tests etc.
As far as explanations of why and how acupuncture works (if it does) we are also at a loss.
There are volumes written about acupuncture practices relative to various ailments, and almost all of these treatises talks about meridians and acupuncture points, but I still haven't seen any (scientific) attempt to explain why the insertion of a needle in one of these points should have any kind of effect.
I, personally, have no belief, one way or another, whether or not meridians and acupuncture points are scientific facts. But let's just assume they are, and that they represent especially sensitive zones of the body.
Can we then think of a way a needle in such a point might increase the possibility of interacting with the surrounding atmosphere?
John Wetling, who has worked with ion therapy and acupuncture for years, recently put this question to me. He also suggested that it might have something to do with ions and electric fields.

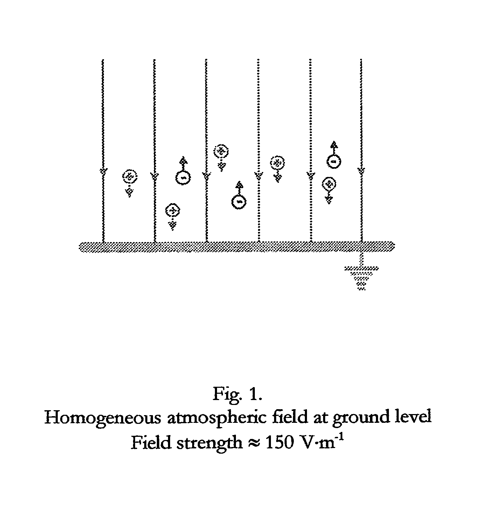

Being a skeptic and a doubter in all non-proven scientific matters, I redefined the question to the following:
**Is there any way a metallic needle inserted subcutaneously in the skin can interact physically with the environment ?**
And I think there is. And in order to explain that we have to look a little at the fascinating topic of atmospheric electricity.
In the outdoor atmosphere there will always be an electric field.
Under fair-weather conditions the field will be directed towards ground, and at flat horizontal areas the field will be more or less homogeneous and have a value about 150 V·m⁻¹, Fig. 1.
The origin of the atmospheric electric field is thunderstorms. At any given time about 2000 thunderstorms are active, creating a voltage difference of about 300,000 volt, between the lower part of the ionosphere and the ground.

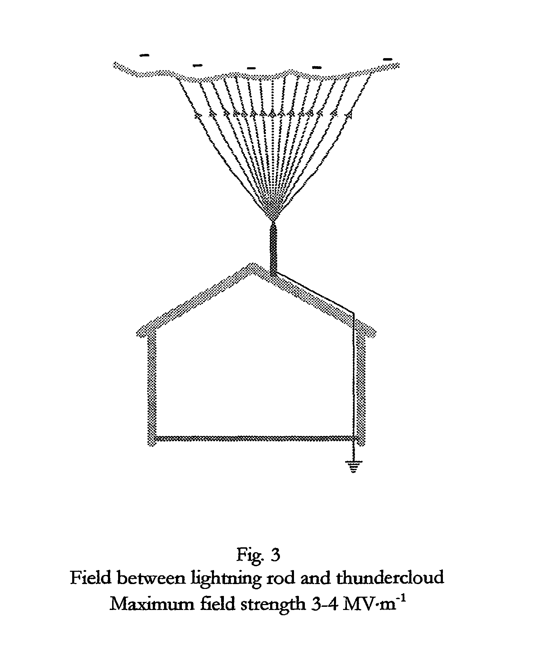

The field will make airborne, charged particles move, the positive particles in the direction of the field, the negative in the opposite direction.
The most important of the charged particles are the atmospheric ions (see for instance *Niels Jonassen: IONS, Compliance Engineering, May*/*June 1999).*
The field-induced motion of the positive ions constitutes a current to ground with an average value of about 3 pA-m⁻² (3.10⁻¹² ampere per square meter).
If now the surface considered is not horizontal the situation changes dramatically.

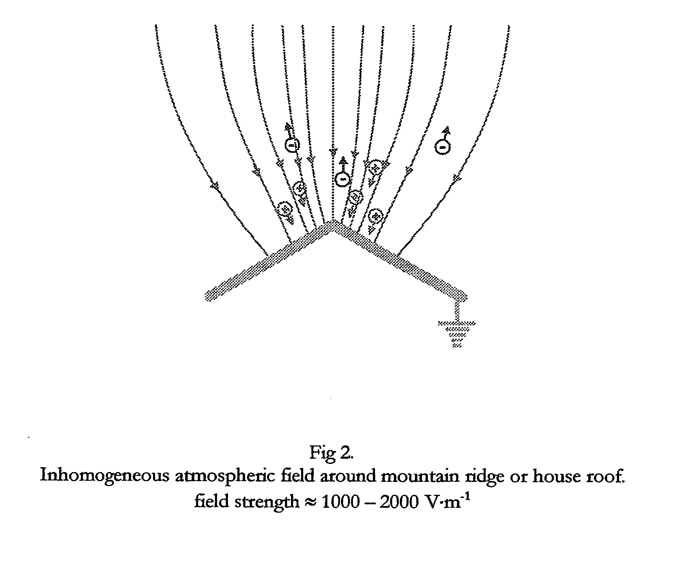

In Fig. 2 is shown the field around a sharp structure, like a mountain ridge or maybe just a house roof.
The field will no longer be homogeneous, but is said to be distorted, having values in the order of 1000-2000 V-m⁻¹, i.e. about 10 times the values at a horizontal surface. Consequently the current to ground will be about 10 times as dense.
A special case of a strongly distorted field is shown in Fig. 3.
Here we have a house supplied with a (grounded) lightning rod. Above the house is a thundercloud with a negative base.
We notice that the direction of the field lines is the opposite of that in fair-weather conditions, as shown in Figs. 1 and 2.
The field strength has its highest value at the tip of the rod, and if the field strength here exceeds a critical value, the break-down field strength, of 3-4 MV·m⁻¹ (3-4 million volt per meter) an electric discharge takes place.
The discharge may take the form of a silent corona discharge or it may start a violent and dramatic lightning discharge.
The field strengths and currents will be much larger than with fair-weather conditions, but the point is, that in both cases a distortion of the electric field, caused by a sharp or pointed "electrode" will increase the current from the atmosphere to the electrode.
And here we are finally approaching the topic of acupuncture.

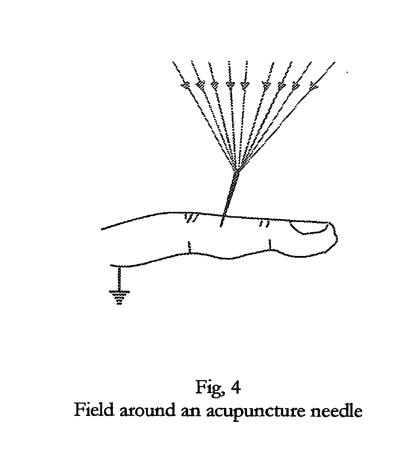

In Fig. 4 is shown a finger with an acupuncture needle inserted.
The finger and the rest of the body are assumed to be grounded and placed in a positive field, i.e. a field directed towards any grounded objects. In the absence of the needle the current to the finger, caused by the field will be distributed more or less evenly over the finger.

The needle, however, will distort the field and concentrate the current to the needle and hence to the point where the needle is inserted.
Now the question obviously arises: What is the probability for the body to be in an electric field ? First of all it should be stressed, that the outdoor atmospheric electric field, discussed in Figs. 1, 2, and 3, is shielded almost one hundred percent by most building materials.
On the other hand (weak) electric fields are almost always present, even in indoor environments, originating from people moving or insulating materials being acciden-tally charged.
And at the same time there are always ions present in the atmosphere, originating primarily from decay of naturally occurring airborne nuclides.
As a consequence weak currents will constantly be flowing to the body, the direction depending on the origin of the fields in the environment.
As explained above the insertion of needles in the skin may increase the currents to selected parts of the body, and it is obvious that it is possible to increase the magnitude of the fields as well as of the ion concentrations and thus of the resulting currents by using well-established principles of physics.
This is for instance done with the so-called 3A-therapy developed by John Wetling.

The present little dissertation has demonstrated that basic principles of Physics predict that the subcutaneous insertion of needles in the skin may increase the transport of charged particles to the point of insertion.
And that is all.
To repeat:
The author takes no stand to the question of meridians and acupuncture points or more generally to the possible effects of acupuncture.
But I believe that the practitioners of acupuncture and related practices may gain a little more insight in the physical background of their work by applying the principles presented above.

### Special effects of the invention

According to the invention the claim is that the insertion of an acupuncture needle in the skin may have two types of physical effects, that is 1) the needle will concentrate a possible electrical field and hence enhance the flow of ions to the body and 2) that it is the current delivered by the neutralized ions, which is the cause of the acupunctural effect.
It appears from the application in question that it is of vital importance that the electrical potential of the object being treated, human or animal, relative to ground is being monitored continuously. This is a question, which never seems to have been addresses in traditional acupuncture therapy, and this may be an essential reason for the great variance in the effects of traditional acupuncture treatment.
The invention is clearly based on the concept of acupuncture. But where traditional acupuncture relies on more or less odd atmospheric electrical conditions, and on the insertion of needles in discrete points, with this invention it is possible to spray a larger area with ions, and accurately to control and measure the exposure (number of ions per unit time) as well as the total dose (total passed charge).

### Technical means.

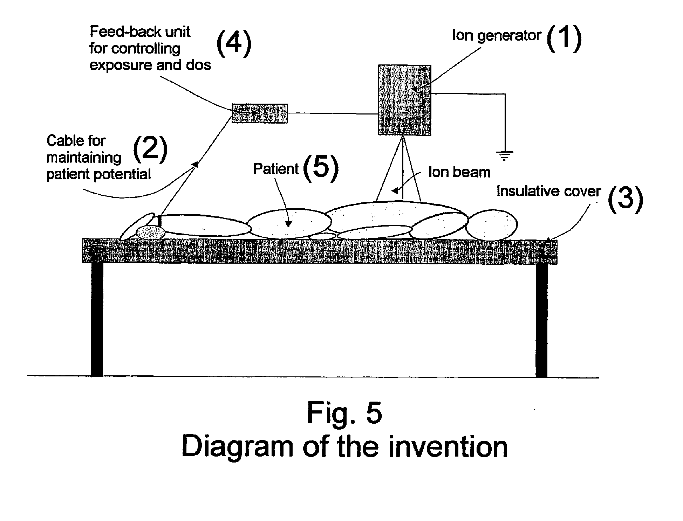

The invention comprises, according to Fig. 5, of a negative ion generator, a feed back unit for measuring and controlling the exposure and dose to the object to be treated, a cable for maintaining the potential of the object and an insulative cover.
By using the invention any person, treating human beings or animals, may, even without a special knowledge of acupuncture, be able to conduct well-controlled and measurable treatments with air ions with effects, which are directly comparable to those of acupuncture.

## Claims

1. An atmospheric electric acupuncture device comprising a negative ion generator (1), electrical cables (2) to maintain the potential of an object (5) to be treated, a back feed unit (4) to control the exposure and dose to the object being treated and an insulating cover (3).

2. A device according to claim 1, wherein the feedback unit is adapted to monitor a number of discharged ions per unit of a time measure.

3. A device according to claims 1 or 2, wherein the feedback unit is adapted to monitor a total charge delivered to the object.

4. A device according to any of the preceding claims, wherein the feedback unit is adapted to monitor a time measure for an exposure of ions.

5. A device according to any of the preceding claims, wherein the feedback unit is adapted to keep the object at a specific potential.

6. A device according to any of the preceding claims, wherein the insulating cover is adapted to carry the object in a horizontal or vertical orientation.

## Patentansprüche

1. Atmosphärisch elektrische Akupunkturvorrichtung mit einem Generator (1) für negative Ionen, mit elektrischen Kabeln (2) zum Aufrechterhalten des Potentials an einem zu behandelnden Objekt (5), mit einer Rückkopplungseinheit (4) zum Steuern der Exposition des für das zu behandelnde Objekt und die Dosierung für das zu behandelnde Objekt und mit einer isolierenden Abdeckung (3).

2. Vorrichtung nach Anspruch 1, bei der die Rückkopplungseinheit dazu eingerichtet ist, eine Anzahl von abgegebenen Ionen pro gemessener Zeiteinheit anzuzeigen.

3. Vorrichtung nach Anspruch 1 oder 2, bei der die Rückkopplungseinheit dazu eingerichtet ist, die gesamte dem Objekt zugeführte Ladung anzuzeigen.

4. Vorrichtung nach einem der vorangehenden Ansprüche, bei der die Rückkopplungseinheit dazu eingerichtet ist, eine Zeitmessung für eine Exposition von Ionen anzuzeigen.

5. Vorrichtung nach einem der vorangehenden Ansprüche, bei der die Rückkopplungseinheit dazu eingerichtet ist, das Objekt bei einem bestimmten Potential zu halten.

6. Vorrichtung nach einem der vorangehenden Ansprüche, bei der die isolierende Abdeckung dazu eingerichtet ist, das Objekt in einer horizontalen oder in einer vertikalen Ausrichtung zu tragen.

## Revendications

1. Un dispositif d'acupuncture à électricité atmosphérique comprenant un générateur d'ions négatifs (1), des câbles électriques (2) pour maintenir le potentiel d'un objet (5) à traiter, une unité de contrôle de retour (4) pour contrôler l'exposition et la dose administrée à l'objet à traiter et un revêtement isolant (3).

2. Un dispositif selon la revendication 1, dans lequel l'unité de contrôle de retour est adaptée pour surveiller le nombre d'ions déchargés par unité de temps.

3. Un dispositif selon la revendication 1 ou 2, dans lequel l'unité de contrôle de retour est adaptée pour surveiller la charge totale délivrée à l'objet.

4. Un dispositif selon l'une quelconque des revendications précédentes, dans lequel l'unité de contrôle de retour est adaptée pour surveiller la durée d'une exposition aux ions.

5. Un dispositif selon l'une quelconque des revendications précédentes, dans lequel l'unité de contrôle de retour est adaptée pour maintenir l'objet à un potentiel spécifique.

6. Un dispositif selon l'une quelconque des revendications précédentes, dans lequel le revêtement isolant est adapté pour transporter l'objet horizontalement ou verticalement.
